# EUROPÄISCHE PATENTSCHRIFT

(11) **EP 2 931 227 B1**
(45) Veröffentlichungstag und Bekanntmachung des Hinweises auf die Patenterteilung: **16.08.2017**
(21) Anmeldenummer: 13799307.7
(22) Anmeldetag: 04.12.2013
(51) Int. Cl.: A61Q 5/10, A61Q 5/08, A61K 8/22, A61K 8/23, A61K 8/24, A61K 8/34, A61K 8/37, C08G 65/26, C08L 71/02

(54) **REDUZIERUNG DES AMMONIAKGERUCHS IN MITTELN ZUM BLONDIEREN UND/ODER BLEICHEN VON KERATINISCHEN FASERN**
REDUCTION OF AMMONIA ODOR IN AGENTS FOR DYEING AND/OR BLEACHING KERATIN FIBERS
RÉDUCTION DE L'ODEUR D'AMMONIAC DANS DES AGENTS DESTINÉS À BLONDIR ET/OU ECLAIRCIR DES FIBRES KÉRATINIQUES

(30) Priorität: 14.12.2012 DE 102012223202
(43) Veröffentlichungstag der Anmeldung: 21.10.2015
(73) Patentinhaber: Henkel AG & Co. KGaA, 40589 Düsseldorf (DE)
(72) Erfinder: NEUBA, Constanze, 41516 Grevenbroich (DE); JANSSEN, Frank, 51103 Köln (DE)
(86) Internationale Anmeldenummer: PCT/EP2013/075469
(87) Internationale Veröffentlichungsnummer: WO 2014/090645

(56) Entgegenhaltungen:
- EP-A2- 2 198 923
- WO-A1-2011/121010
- JP-A- 2003 034 620
- JP-A- 2008 201 727

## Beschreibung

Die vorliegende Erfindung liegt im Gebiet der Kosmetik und betrifft Mittel zum Blondieren und/oder Bleichen von keratinischen Fasern, insbesondere menschlichen Haaren, enthaltend in einem kosmetischen Träger spezielle Kombinationen aus mindestens einem langkettigen Fettalkohol, mindestens einem Glycerylfettsäureester und Ammoniumpersulfat. Zum Blondieren bzw. Bleichen menschlicher Haare werden üblicherweise feste oder pastenförmige Zubereitungen mit festen Oxidationsmitteln unmittelbar vor der Anwendung mit einer verdünnten Wasserstoffperoxid-Lösung vermischt. Diese Mischung wird dann auf das Haar aufgebracht und nach einer bestimmten Einwirkzeit wieder ausgespült. Um eine ausreichende Blondierwirkung zu erzielen, sind derartige Mittel üblicherweise stark alkalisch eingestellt, der pH-Wert liegt hierbei üblicherweise zwischen 9 und 11,5. Derart hohe pH-Werte sind erforderlich, um eine Öffnung der äußeren Schuppenschicht des Haares (Cuticula) zu gewährleisten und somit eine Penetration der Aktiven Spezies (Oxidationsmittel) ins Haar zu ermöglichen. Siehe JP2003034620 und JP2008201727. Zur Erzielung moderater Aufhelleffekte ist Wasserstoffperoxid das Oxidationsmittel der Wahl. Wird jedoch eine stärkere Aufhellung bzw. Blondierung gewünscht, wird Wasserstoffperoxid zusammen mit stärkeren Oxidationsmitteln wie beispielsweise Persulfaten (Natriumpersulfat, Kaliumpersulfat oder Ammoniumpersulfat) eingesetzt.

Aufgrund seiner guten Wasserlöslichkeit hat sich insbesondere der Einsatz von Ammoniumpersulfat in Blondiermitteln als vorteilhaft erwiesen. Darüber hinaus wird bei der Auflösung von Ammoniumpersulfat in alkalischem Milieu Ammoniak freigesetzt. Ammoniak dient - neben seiner Wirkung als Alkalisierungsmittel - zusätzlich der Quellung der Haare und als Penetrations- bzw. Penetrationshilfsmittel für die für den Blondiervorgang verantwortlichen Spezies (wie Persulfat und Wasserstoffperoxid).

Der Nachteil des Einsatzes von Ammoniumpersulfat liegt in seinem auf der Freisetzung des Ammoniaks beruhenden, intensiven, stechenden Geruch. Die mit dem Einsatz von Ammoniumpersulfat verbundenen anwendungstechnischen Vorteile sind jedoch so vielfältig, dass das Ammoniumpersulfat trotz seines unangenehmen, stechenden Geruchs in einer Vielzahl handelsüblicher oxidativer Blondier- bzw. Bleichmittel verwendet wird.

Bislang hat es nicht an Bestrebungen gefehlt, den Ammoniak-Geruch in Blondiermitteln zu überdecken. Insbesondere die dauerhafte Geruchsminimierung über die gesamte Anwendungsdauer ist jedoch nur sehr schwierig zu erreichen. Die Zeitspanne, innerhalb der sich der Anwender von Blondiermitteln in Kontakt mit dem Blondiermittel befindet, reicht von der Herstellung der Anwendungsmischung über deren Auftragen auf die Haare und den Zeitraum des Einwirkens bis zum Auswaschen der Formulierung. Bei üblichen Einwirkzeiten von 30 bis 45 Minuten kann der gesamte Prozess bis zu 90 Minuten, im Höchstfall bis zu zwei Stunden Zeit in Anspruch nehmen. Eine geruchliche Abdeckung des Ammoniaks, die über diesen gesamten Zeitraum wirksam ist, stellt die höchste Herausforderung dar. Gerade auf diesem Feld besteht noch starker Optimierungsbedarf, und eine optimale Möglichkeit zur dauerhaften Reduktion des Ammoniak-Geruchs ist aus dem Stand der Technik bislang nicht bekannt.

Aufgabe der vorliegenden Erfindung war daher die Bereitstellung von Blondiermitteln auf Basis der Oxidationsmittelkombination Ammoniumpersulfat/Wasserstoffperoxid, die einen reduzierten Ammoniakgeruch besitzen. Im Hinblick auf ihre Aufhellwirkung sollen die geruchsoptimierten Mittel keine Einbußen erleiden.

Es war hierbei insbesondere die Aufgabe der vorliegenden Erfindung, über die gesamte Anwendungsdauer eine Reduktion des Ammoniakgeruchs zu erzielen. Auch nach maximal zweistündigem Kontakt sollte die geruchliche Wahrnehmung des Ammoniaks immer noch wirksam minimiert sein.

Überraschenderweise hat sich im Zuge der zu dieser Erfindung führenden Arbeiten herausgestellt, dass es möglich ist, die geruchliche Wahrnehmung von Ammoniak in Mitteln zum Blondieren und/oder Bleichen von keratinischen Fasern wirksam über den gesamten Anwendungszeitraum zu minimieren, wenn den Mitteln neben Ammoniumpersulfaten bestimmte langkettige Fettalkohole und bestimmte Glycerinfettsäureester zugesetzt werden. Offenbart wird hier ein Mittel zum Blondieren und/oder Bleichen von keratinischen Fasern, insbesondere menschlichen Haaren, enthaltend in einem kosmetischen Träger
(a) mindestens einen Fettalkohol ausgewählt aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und Brassidylalkohol ((13*E*)-Docosen-1-ol),
(b) Ammoniumpersulfat sowie gegebenenfalls ein weiteres Persulfatsalz,
(c) mindestens einen Glycerylfettsäureester der allgemeinen Formel (I), 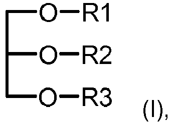 worin R1, R2 und R3 unabhängig voneinander für ein Wasserstoffatom oder eine Gruppierung der Formel (II) stehen, 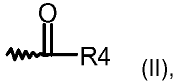 worin
   R4 für eine unverzweigte oder verzweigte, gesättige oder ungesättigte C₁₁-C₂₇-Alkylgruppe steht,
   mit der Maßgabe, dass mindestens einer und maximal zwei der Reste ausgewählt aus R1, R2 und R3 für eine Gruppierung der Formel (II) stehen.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare. Unter dem erfindungsgemäß verwendeten Begriff "Mittel zum Blondieren und/oder Bleichen" von Keratinfasern werden oxidative Entfärbemittel verstanden, die auf Basis von Oxidationsmittelkombinationen aus Wasserstoffperoxid und Persulfaten arbeiten. Entsprechende Blondier- bzw. Bleichmittel werden nicht zur Erzielung moderater Bleicheffekte, sondern zur Erzielung möglichst starker Bleich- bzw. Blondiereffekte eingesetzt.

Die erfindungsgemäßen Mittel enthalten die erfindungswesentlichen Bestandteile in einem kosmetischen Träger, bevorzugt in einem geeigneten wässrigen, alkoholischen oder wässrigalkoholischen Träger. Zum Zwecke der Blondierung können solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind, sein.

Bevorzugt kommen die erfindungsgemäßen Blondiermittel in Form eines auf 2 Komponenten basierenden Blondiersystems auf den Markt. Hierbei handelt es sich bei der ersten Komponente (Zubereitung A) um eine flüssige, gel- oder cremeförmige Komponente, welche Wasserstoffperoxid in dem zuvor beschriebenen kosmetischen Träger enthält. Bei der zweiten Komponente handelt es sich bevorzugt um eine feste, granulatförmige, oder pastöse Zubereitung (Zubereitung B), welche die Persulfate bzw. die speziellen Persulfatgemische beinhaltet. Kurz vor der Anwendung werden die Zubereitung A und die Zubereitung B zur Herstellung des anwendungsbereiten Blondiermittels miteinander vermischt.

Als ersten wesentlichen Formulierungsbestandteil (a) enthalten die Mittel zum Blondieren und/oder Bleichen von keratinischen Fasern mindestens einen langkettigen Fettalkohol (a), der aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und Brassidylalkohol ((13*E*)-Docosen-1-ol) ausgewählt ist.

Diese langkettigen Fettalkohole (a) besitzen ein Kettenlänge von mindestens 20 C-Atomen. Innerhalb dieser Gruppe haben sich spezielle langkettige Fettalkohole im Hinblick auf die geruchliche Optimierung der Blondierformulierungen als ganz besonders geeignet erwiesen. Bevorzugt ist das Mittel zum Blondieren und/oder Bleichen von keratinischen Fasern dadurch gekennzeichnet, dass es Behenylalkohol (Docosan-1-ol) enthält.

In einer weiteren besonders bevorzugten Ausführungsform ist ein Mittel zum Blondieren und/oder Bleichen von keratinischen Fasern dadurch gekennzeichnet, dass es Arachylalkohol (Eicosan-1-ol) und Behenylalkohol (Docosan-1-ol) enthält. Weiterhin hat sich herausgestellt, dass es von Vorteil ist, wenn die langkettigen Fettalkohole (a), insbesondere Arachylalkohol (Eicosan-1-ol) und/oder Behenylalkohol (Docosan-1-ol), in bestimmten Mengenbereichen enthalten sind. Hierbei hat es sich als besonders bevorzugt herausgestellt, wenn die Gesamtmenge der Fettalkohole (a) ausreichend hoch gewählt wird, so dass eine effektive Reduktion des Ammoniakgeruchs erzielt werden kann. Auf der anderen Seite haben sich zu hohe Einsatzmengen an Fettalkoholen (a) als nachteilig erwiesen, da das Blondiermittel in letzterem Fall zu stark verdickt wird. Bedingt durch die zu hohe Viskosität wird die Diffusion der aktiven Spezies (Oxidationsmittel) in den Haarschaft hinein verhindert, was in einer Verschlechterung der Blondierleistung resultiert.

Auf Basis dieser Befunde hat es sich als besonders vorteilhaft herausgestellt, wenn das erfindungsgemäße Mittel ein oder mehrere langkettige Fettalkohole (a) aus der Gruppe Behenylalkohol (Docosan-1-ol), Erucylalkohol ((13*Z*)-Docos-13-en-1-ol) und Brassidylalkohol ((13*E*)-Docosen-1-ol) in einer Gesamtmenge von 0,3 bis 3,4 Gew.-%, bevorzugt von 0,4 bis 2,6 Gew.-%, weiter bevorzugt von 0,5 bis 1,8 Gew.-% und besonders bevorzugt von 0,6 bis 0,9 Gew.-% - bezogen auf das Gesamtgewicht des anwendungsbereiten Mittels - enthält.

In einer ganz besonders bevorzugten Ausführungsform ist ein erfindungsgemäßes Mittel dadurch gekennzeichnet, dass es als Fettalkohol(e) (a) Arachylalkohol (Eicosan-1-ol) und Behenylalkohol (Docosan-1-ol) in einer Gesamtmenge von 0,3 bis 3,4 Gew.-%, bevorzugt von 0,4 bis 2,6 Gew.-%, weiter bevorzugt von 0,5 bis 1,8 Gew.-% und besonders bevorzugt von 0,6 bis 0,9 Gew.-% - bezogen auf das Gesamtgewicht des anwendungsbereiten Mittels - enthält. Neben den speziellen langkettigen Fettalkoholen (a) mit einer Kettenlänge von mindestens 20 C-Atomen kann das Mittel zusätzlich auch noch weitere, kürzerkettige Fettalkohole mit einer Kettenlänge von 12 bis 18 C-Atomen enthalten. Geeignete kürzerkettige Fettalkohole mit einer gesättigten C₁₂-C₁₈-Alkylkette sind beispielsweise Dodecan-1-ol (Dodecylalkohol, Laurylalkohol), Tetradecan-1-ol (Tetradecylalkohol, Myristylalkohol), Hexadecan-1-ol (Hexadecylalkohol, Cetylalkohol, Palmitylalkohol) und Octadecan-1-ol (Octadecylalkohol, Stearylalkohol). Ein geeigneter kürzerkettiger Fettalkohol mit einer ungesättigten C₁₂-C₁₈-Alkylkette ist beispielsweise (9*Z*)-Octadec-9-en-1-ol (Oleylalkohol).

Um die erfindungsgemäßen Mittel jedoch nicht zu stark zu verdicken und hierdurch bedingt die Blondierleistung zu verschlechtern, liegt die Gesamtmenge aller im Mittel enthaltenenen Fettalkohole jedoch unterhalb bestimmter Gewichtsgrenzen.

In einer weiteren besonders bevorzugten Ausführungsform ist ein erfindungsgemäßes Mittel daher dadurch gekennzeichnet, dass die Gesamtmenge aller im Mittel enthaltenen Fettalkohole - bezogen auf das Gesamtgewicht des anwendungsbereiten Mittels - eine Menge von 5,5 Gew.-%, bevorzugt eine Menge von 4,8 Gew.-%, weiter bevorzugt eine Menge von 4,1 Gew.-% und besonders bevorzugt eine Menge von 3,5 Gew.-% nicht überschreitet.

Unter der Gesamtmenge aller im anwendungsbereiten Mittel enthaltenene Fettalkohole wird in diesem Zusammenhang die Gesamtheit aller Fettalkohole, d.h. die Gesamtheit aller Alkanole mit einer Kettenlänge von 8 bis 40 C-Atomen, verstanden. Der Berechnungsgrundlage für den in Gew.-% angegebenen Mengenanteil ist hierbei das Gesamtgewicht des anwendungsbereiten Mittels.

Als zweiten wesentlichen Formulierungsbestandteil (b) enthalten die erfindungsgemäßen Mittel zum Blondieren und/oder Bleichen von keratinischen Fasern (b) Ammoniumpersulfat sowie gegebenenfalls ein weiteres Persulfatsalz.

Unter Ammoniumpersulfat wird im Sinne der vorliegenden Erfindung das Persufat mit der Summenfomel (NH₄)₂S₂O₈ verstanden. Weitere geeignete Persulfatsalze sind beispielsweise Kaliumpersulfat (Summenformel K₂S₂O₈) und Natriumpersulfat (Summenformel Na₂S₂O₈).

Im Zuge der zu dieser Erfindung führenden Arbeiten hat es sich sowohl im Hinblick auf die Blondierleistung als auch im Hinblick auf die Minimierung des Ammoniak-Geruchs als optimal herausgestellt, wenn die Mittel nicht Ammoniumpersulfat allein, sondern ein Gemisch aus Ammoniumpersulfat und Kaliumpersulfat enthalten.

In einer weiteren bevorzugten Ausführungsform ist ein erfindungsgemäßes Mittel daher dadurch gekennzeichnet, dass es Ammoniumpersulfat und Kaliumpersulfat enthält. Darüber hinaus ist es bevorzugt, wenn das bzw. die Persulfate in bestimmten Mengenbereichen im erfindungsgemäßen Mittel enthalten sind.

In einer weiteren bevorzugten Ausführungsform ist ein erfindungsgemäßes Mittel daher dadurch gekennzeichnet, dass es Ammoniumpersulfat und gegebenenfalls weitere Persulfate in einer Gesamtmenge von 10 bis 18 Gew.-%, bevorzugt von 11 bis 17 Gew.-%, weiter bevorzugt von 12 bis 16 Gew.-% und besonders bevorzugt von 13 bis 15 Gew.-% - bezogen auf das Gesamtgewicht des anwendungsbereiten Mittels - enthält.

In einer ganz besonders bevorzugten Ausführungsform ist ein erfindungsgemäßes Mittel dadurch gekennzeichnet, dass es als Persulfatsalze (b) Kaliumpersulfat und Ammoniumpersulfat in einer Gesamtmenge von 10 bis 18 Gew.-%, bevorzugt von 11 bis 17 Gew.-%, weiter bevorzugt von 12 bis 16 Gew.-% und besonders bevorzugt von 13 bis 15 Gew.-% - bezogen auf das Gesamtgewicht des anwendungsbereiten Mittels - enthält. Weiterhin kann die geruchliche Wahrnehmung des Ammoniaks ohne Einbußen der Blondierleistung besonders effektiv und über einen besonders langen Zeitraum minimiert werden, wenn Kaliumpersulfat und Ammoniumperulfat in bestimmten Mengenverhältnissen zueinander eingesetzt werden. Die angegebenen Mengenverhältnisse beziehen sich hierbei auf die Einsatzmengen beider Persulfate im anwendungsbereiten Mittel.

Bevorzugt werden Kaliumpersulfat zu Ammoniumpersulfat im in einem Gewichtsverhältnis von 1:1 bis 6:1 zueinander eingesetzt. Weiter bevorzugt werden Kaliumpersulfat zu Ammoniumpersulfat im in einem Gewichtsverhältnis von 2:1 bis 5:1 zueinander eingesetzt. Ganz besonders bevorzugt werden Kaliumpersulfat zu Ammoniumpersulfat im in einem Gewichtsverhältnis von 3:1 bis 4:1 zueinander eingesetzt. Dementsprechend ist ein besonders bevorzugtes Mittel dadurch gekennzeichnet, dass es Kaliumpersulfat und Ammoniumpersulfat enthält, wobei die Einsatzmenge des Kaliumpersulfats im andwendungsbereiten Mittel drei- bis viermal so hoch ist wie die Einsatzmenge des Ammoniumpersulfats.

In einer weiteren ganz besonders bevorzugten Ausführungsform ist ein erfindungsgemäßes Mittel dadurch gekennzeichnet, dass es als Persulfatsalze (b) Kaliumpersulfat und Ammoniumpersulfat enthält, wobei das Gewichtsverhältnis von Kaliumpersulfat zu Ammoniumpersulfat im Bereich von 1:1 bis 6:1, bevorzugt von 2:1 bis 5:1 und besonders bevorzugt von 3:1 bis 4:1 - bezogen auf das Gesamtgewicht des anwendungsbereiten Mittels - liegt.

Als dritten wesentlichen Formulierungsbestandteil (c) enthalten die erfindungsgemäßen Mittel mindestens einen Glycerylfettsäureester der allgemeinen Formel (I), 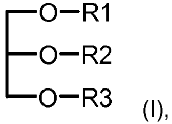 worin
R1, R2 und R3 unabhängig voneinander für ein Wasserstoffatom oder eine Gruppierung der Formel (II) stehen, 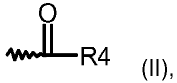 worin
R4 für eine unverzweigte oder verzweigte, gesättige oder ungesättigte C₁₁-C₂₇-Alkylgruppe steht,
mit der Maßgabe, dass mindestens einer und maximal zwei der Reste ausgewählt aus R1, R2 und R3 für eine Gruppierung der Formel (II) stehen.

Der Rest R4 in der Formel (II) steht für eine unverzweigte oder verzweigte, gesättige oder ungesättigte C₁₁-C₂₇-Alkylgruppe.

Bevorzugt steht R4 für eine unverzweigte, gesättigte C₁₁-C₂₇-Alkylgruppe. Weiter bevorzugt steht R4 für eine unverzweigte, gesättigte C₁₃-C₂₃-Alkylgruppe. Besonders bevorzugt steht R4 für eine unverzweigte, gesättigte C₁₅-C₁₇-Alkylgruppe

In einer weiteren besonders bevorzugten Ausführungsform ist ein erfindungsgemäßes Mittel dadurch gekennzeichnet, dass es als Glycerylfettsäureester (c) der allgemeinen Formel (I) mindestens eine Verbindung aus der Gruppe der Formeln (la) bis (Id) enthält.

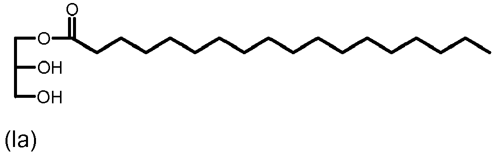

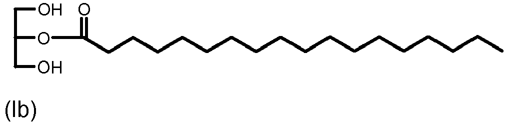

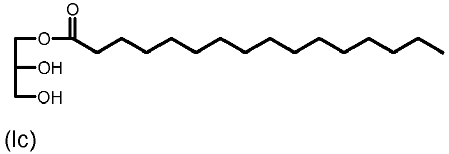

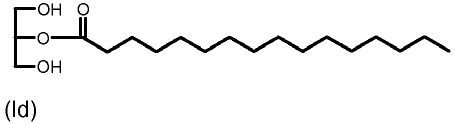

Die Verbindungen der Formeln (Ia) bis (Id) sind auch unter den Namen Glycerylmonostearat und Glycerylmonopalmitat bekannt.

In einer weiteren besonders bevorzugten Ausführungsform ist ein erfindungsgemäßes Mittel dadurch gekennzeichnet, dass es als Glycerylfettsäureester (c) der allgemeinen Formel (I) mindestens eine Verbindung aus der Gruppe der Formeln (Ie) bis (Ih) enthält.

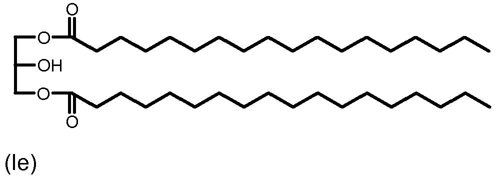

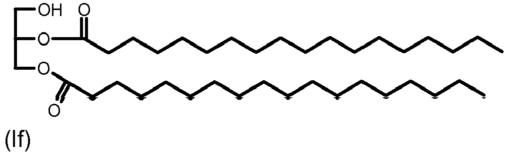

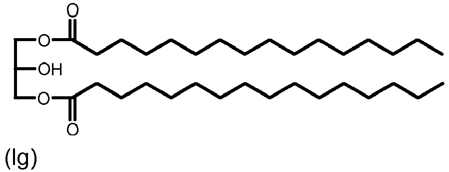

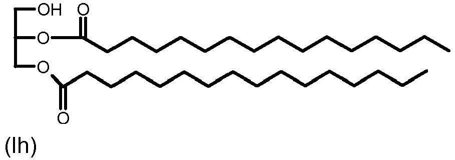

Die Verbindungen der Formel (Ie) bis (Ih) sind auch unter den Namen Glyceryldistearat und Glyceryldipalmitat bekannt.

In einer ganz besonders bevorzugten Ausführungsform ist ein erfindungsgemäßes Mittel dadurch gekennzeichnet, dass es als Glycerylfettsäureester (c) der allgemeinen Formel (I) mindestens eine Verbindung ausgewählt aus den Formeln (la) bis (Ih) enthält.

In einer weiteren ganz besonders bevorzugten Ausführungsform ist ein erfindungsgemäßes Mittel dadurch gekennzeichnet, dass es als Glycerylfettsäureester (c) der allgemeinen Formel (I) mindestens eine Verbindung ausgewählt aus Glycerylmonostearat, Glyceryldistearat, Glycerylmonopalmitat und Glyceryldipalmitat enthält.

Die Glycerylfettsäureester (c) bewirken in Kombination mit den langkettigen Fettalkoholen (a) eine Reduktion des Ammoniak-Geruchs. Die Geruchsreduktion ist besonders ausgeprägt, wenn Glycerylfettsäureester (c) und langkettige Fettalkohole (a) in bestimmten Verhältnissen zueinander eingesetzt werden. Daher sind auch die Glycerylfettsäureester (c) bevorzugt in bestimmten Mengen im erfindungsgemäßen Mittel enthalten.

Besonders bevorzugt ist es, wenn im erfindungsgemäßen Mittel ein oder mehrere Glycerylfettsäureester (c) in einer Gesamtmenge von 0,1 bis 1,8 Gew.-%, bevorzugt von 0,2 bis 1,3 Gew.-%, weiter bevorzugt von 0,25 bis 0,8 Gew.-% und besonders bevorzugt von 0,3 bis 0,6 Gew.-% - bezogen auf das Gesamtgewicht des anwendungsbereiten Mittels - enthalten sind.

In einer weiteren explizit ganz besonders bevorzugten Ausführungsform ist ein erfindungsgemäßes Mittel dadurch gekennzeichnet, dass es als Glycerylfettsäureester (c) der allgemeinen Formel (I) mindestens eine Verbindung ausgewählt aus Glycerylmonostearat, Glyceryldistearat, Glycerylmonopalmitat und Glyceryldipalmitat in einer Gesamtmenge von 0,1 bis 1,8 Gew.-%, bevorzugt von 0,2 bis 1,3 Gew.-%, weiter bevorzugt von 0,25 bis 0,8 Gew.-% und besonders bevorzugt von 0,3 bis 0,6 Gew.-% - bezogen auf das Gesamtgewicht des anwendungsbereiten Mittels - enthält. Ein bevorzugtes Mittel ist dadurch gekennzeichnet, dass es - bezogen auf das Gesamtgewicht des anwendungsbereiten Mittels -
(a) 0,4 bis 2,6 Gew.-% Behenylalkohol (Docosan-1-ol)
(b) Kaliumpersulfat und Ammoniumpersulfat in einer Gesamtmenge von 10 bis 18 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,1 bis 1,8 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,6 bis 0,9 Gew.-% Behenylalkohol (Docosan-1-ol)
(b) Kaliumpersulfat und Ammoniumpersulfat in einer Gesamtmenge von 10 bis 18 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,1 bis 1,8 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,4 bis 2,6 Gew.-% Behenylalkohol (Docosan-1-ol)
(b) Kaliumpersulfat und Ammoniumpersulfat in einer Gesamtmenge von 10 bis 18 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,2 bis 1,3 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,6 bis 0,9 Gew.-% Behenylalkohol (Docosan-1-ol)
(b) Kaliumpersulfat und Ammoniumpersulfat in einer Gesamtmenge von 10 bis 18 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,2 bis 1,3 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,4 bis 2,6 Gew.-% Behenylalkohol (Docosan-1-ol)
(b) Kaliumpersulfat und Ammoniumpersulfat in einer Gesamtmenge von 10 bis 18 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,25 bis 0,8 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,6 bis 0,9 Gew.-% Behenylalkohol (Docosan-1-ol)
(b) Kaliumpersulfat und Ammoniumpersulfat in einer Gesamtmenge von 10 bis 18 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,25 bis 0,8 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,4 bis 2,6 Gew.-% Behenylalkohol (Docosan-1-ol)
(b) Kaliumpersulfat und Ammoniumpersulfat in einer Gesamtmenge von 10 bis 18 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,3 bis 0,6 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,6 bis 0,9 Gew.-% Behenylalkohol (Docosan-1-ol)
(b) Kaliumpersulfat und Ammoniumpersulfat in einer Gesamtmenge von 10 bis 18 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,3 bis 0,6 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,4 bis 2,6 Gew.-% Behenylalkohol (Docosan-1-ol)
(b) Kaliumpersulfat und Ammoniumpersulfat in einer Gesamtmenge von 11 bis 17 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,1 bis 1,8 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,6 bis 0,9 Gew.-% Behenylalkohol (Docosan-1-ol)
(b) Kaliumpersulfat und Ammoniumpersulfat in einer Gesamtmenge von 11 bis 17 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,1 bis 1,8 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,4 bis 2,6 Gew.-% Behenylalkohol (Docosan-1-ol)
(b) Kaliumpersulfat und Ammoniumpersulfat in einer Gesamtmenge von 11 bis 17 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,2 bis 1,3 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,6 bis 0,9 Gew.-% Behenylalkohol (Docosan-1-ol)
(b) Kaliumpersulfat und Ammoniumpersulfat in einer Gesamtmenge von 11 bis 17 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,2 bis 1,3 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,4 bis 2,6 Gew.-% Behenylalkohol (Docosan-1-ol)
(b) Kaliumpersulfat und Ammoniumpersulfat in einer Gesamtmenge von 11 bis 17 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,25 bis 0,8 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,6 bis 0,9 Gew.-% Behenylalkohol (Docosan-1-ol)
(b) Kaliumpersulfat und Ammoniumpersulfat in einer Gesamtmenge von 11 bis 17 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,25 bis 0,8 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,4 bis 2,6 Gew.-% Behenylalkohol (Docosan-1-ol)
(b) Kaliumpersulfat und Ammoniumpersulfat in einer Gesamtmenge von 11 bis 17 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,3 bis 0,6 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,6 bis 0,9 Gew.-% Behenylalkohol (Docosan-1-ol)
(b) Kaliumpersulfat und Ammoniumpersulfat in einer Gesamtmenge von 11 bis 17 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,3 bis 0,6 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,4 bis 2,6 Gew.-% Behenylalkohol (Docosan-1-ol)
(b) Kaliumpersulfat und Ammoniumpersulfat in einer Gesamtmenge von 12 bis 16 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,1 bis 1,8 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,6 bis 0,9 Gew.-% Behenylalkohol (Docosan-1-ol)
(b) Kaliumpersulfat und Ammoniumpersulfat in einer Gesamtmenge von 12 bis 16 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,1 bis 1,8 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,4 bis 2,6 Gew.-% Behenylalkohol (Docosan-1-ol)
(b) Kaliumpersulfat und Ammoniumpersulfat in einer Gesamtmenge von 12 bis 16 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,2 bis 1,3 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,6 bis 0,9 Gew.-% Behenylalkohol (Docosan-1-ol)
(b) Kaliumpersulfat und Ammoniumpersulfat in einer Gesamtmenge von 12 bis 16 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,2 bis 1,3 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,4 bis 2,6 Gew.-% Behenylalkohol (Docosan-1-ol)
(b) Kaliumpersulfat und Ammoniumpersulfat in einer Gesamtmenge von 12 bis 16 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,25 bis 0,8 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,6 bis 0,9 Gew.-% Behenylalkohol (Docosan-1-ol)
(b) Kaliumpersulfat und Ammoniumpersulfat in einer Gesamtmenge von 12 bis 16 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,25 bis 0,8 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,4 bis 2,6 Gew.-% Behenylalkohol (Docosan-1-ol)
(b) Kaliumpersulfat und Ammoniumpersulfat in einer Gesamtmenge von 12 bis 16 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,3 bis 0,6 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,6 bis 0,9 Gew.-% Behenylalkohol (Docosan-1-ol)
(b) Kaliumpersulfat und Ammoniumpersulfat in einer Gesamtmenge von 12 bis 16 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,3 bis 0,6 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,4 bis 2,6 Gew.-% Behenylalkohol (Docosan-1-ol)
(b) Kaliumpersulfat und Ammoniumpersulfat in einer Gesamtmenge von 13 bis 15 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,1 bis 1,8 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,6 bis 0,9 Gew.-% Behenylalkohol (Docosan-1-ol)
(b) Kaliumpersulfat und Ammoniumpersulfat in einer Gesamtmenge von 13 bis 15 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,1 bis 1,8 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,4 bis 2,6 Gew.-% Behenylalkohol (Docosan-1-ol)
(b) Kaliumpersulfat und Ammoniumpersulfat in einer Gesamtmenge von 13 bis 15 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,2 bis 1,3 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,5 bis 1,8 Gew.-% Behenylalkohol (Docosan-1-ol)
(b) Kaliumpersulfat und Ammoniumpersulfat in einer Gesamtmenge von 13 bis 15 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,2 bis 1,3 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,3 bis 3,4 Gew.-% Behenylalkohol (Docosan-1-ol)
(b) Kaliumpersulfat und Ammoniumpersulfat in einer Gesamtmenge von 13 bis 15 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,25 bis 0,8 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,6 bis 0,9 Gew.-% Behenylalkohol (Docosan-1-ol)
(b) Kaliumpersulfat und Ammoniumpersulfat in einer Gesamtmenge von 13 bis 15 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,25 bis 0,8 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,3 bis 3,4 Gew.-% Behenylalkohol (Docosan-1-ol)
(b) Kaliumpersulfat und Ammoniumpersulfat in einer Gesamtmenge von 13 bis 15 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,3 bis 0,6 Gew.-% enthält.

Ein weiteres besonders bevorzugtes erfindungsgemäßes Mittel ist dadurch gekennzeichnet, dass es - bezogen auf das Gesamtgewicht des anwendungsbereiten Mittels -
(a) 0,6 bis 0,9 Gew.-% Behenylalkohol (Docosan-1-ol)
(b) Kaliumpersulfat und Ammoniumpersulfat in einer Gesamtmenge von 13 bis 15 Gew.-% und
(c) eine oder mehrere Verbindungen aus der Gruppe Glycerylmonostearat, Glyceryldistearat, Glycerylmonopalmitat und Glyceryldipalmitat in einer Gesamtmenge von 0,3 bis 0,6 Gew.-% enthält.

Im Zuge der zu dieser Erfindung führenden Arbeiten hat sich weiterhin gezeigt, dass die erfindungsgemäße Aufgabenstellung insbesondere dann vollständig und in zufriedenstellender Weise gelöst werden kann, wenn die erfindungsgemäßen Mittel weitere ausgewählte Formulierungsbestandteile enthalten. Besonders bevorzugt enthalten die Mittel zusätzlich mindestens ein oder mehrere amphotere und/oder zwitterionische Tenside. Tenside sind amphiphile (bifunktionelle) Verbindungen, die aus mindestens einem hydrophoben und mindestens einem hydrophilen Molekülteil bestehen. Der hydrophobe Rest ist bevorzugt eine Kohlenwasserstoffkette mit 8-28 Kohlenstoff-Atomen, die gesättigt oder ungesättigt, linear oder verzweigt sein kann. Besonders bevorzugt ist diese C₈-C₂₈-Alkylkette linear.

Basiseigenschaften der Tenside sind die orientierte Absorption an Grenzflächen sowie die Aggregation zu Micellen und die Ausbildung von lyotrophen Phasen.

Die amphoteren Tenside werden in ampholytische Tenside und zwitterionische Tenside unterteilt. Unter ampholytischen Tensiden werden solche oberflächenaktiven Verbindungen verstanden, die sowohl saure (beispielsweise -COOH oder -SO₃H-Gruppen) als auch basische hydrophile Gruppen (beispielsweise Aminogruppen) besitzen und sich also je nach Bedingung sauer oder basisch verhalten. Unter zwitterionischen Tensiden versteht der Fachmann Tenside, die im selben Molekül sowohl eine negative als auch eine positive Ladung tragen.

Beispiele für bevorzugte zwitterionische Tenide sind die Betaine, die N-Alkyl-N,N-dimethyl-ammonium-glycinate, die N-Acyl-aminopropyl-N,N-dimethylammoniumglycinate und die 2-Alkyl-3-carboxymethyl-3-hydroxyethyl-imidazoline mit jeweils 8 bis 24-Catomen in der Alkylgruppe.

Beispiele für bevorzugte ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils 8 bis 24 C-Atomen in der Alkylgruppe.

Zur Lösung der erfindungsgemäßen Aufgabenstellung ganz besonders geeignete amphotere und/oder zwitterionische Tenside sind die unter der INCI-Bezeichnung Disodium Cocoamphodipropionat bekannten Verbindungen.

Zur Lösung der erfindungsgemäßen Aufgabenstellung ganz besonders geeignete amphotere und/oder zwitterionische Tenside sind weiterhin die unter der INCI-Bezeichnung Cocamidopropyl Betain bekannten Verbindungen.

In den erfindungsgemäßen Mitteln sind die amphoteren und/oder zwitterionischen Tenside bevorzugt in speziellen Mengenbereichen enthalten. Bevorzugt enthalten die Mittel zusätzlich ein oder mehrere amphotere und/der zwitterionische Tenside in einer Gesamtmenge von 0,01 bis 0,5 Gew.-%, bevorzugt von 0,03 bis 0,4 Gew.-%, weiter bevorzugt von 0,05 bis 0,3 Gew.-% und besonders bevorzugt von 0,07 bis 0,2 Gew.-% - bezogen auf das Gesamtgewicht des anwendungsbereiten Mittels.

In einer weiteren besonders bevorzugten Ausführungsform ist ein erfindungsgemäßes Mittel dementsprechend dadurch gekennzeichnet, dass es zusätzlich ein oder mehrere amphotere und/oder zwitterionische Tenside in einer Gesamtmenge von 0,01 bis 0,5 Gew.-%, bevorzugt von 0,03 bis 0,4 Gew.-%, weiter bevorzugt von 0,05 bis 0,3 Gew.-% und besonders bevorzugt von 0,07 bis 0,2 Gew.-% - bezogen auf das Gesamtgewicht des anwendungsbereiten Mittels - enthält.

Der Zusatz von einem oder mehreren amphoteren und/oder zwitterionischen Tensiden kann die geruchlichen Eigenschaften der Blondier- bzw. Bleichmittel weiter verbessern.

Überraschenderweise hat sich in diesem Zusammenhang herausgestellt, dass die weitere Minimierung des Ammoniakgeruchs nicht generell bei Zusatz jedes Tensidtyps eintritt. Der Zusatz von anionischen Tensiden verschlechtert überraschenderweise die geruchlichen Eigenschaften der erfindungsgemäßen Mittel wieder, da bei Zusatz von anionischen Tensiden der Ammoniak-Geruch wieder leicht verstärkt wird.

In einer bevorzugten Ausführungsform sind die erfindungsgemäßen Mittel daher frei von anionischen Tensiden. Unter anionischen Tensiden werden in diesem Zusammenhang Tenside mit ausschließlich anionischen Ladungen verstanden.

In einer weiteren besonders bevorzugten Ausführungsform ist ein Mittel zum Färben und/oder Aufhellen von keratinischen Fasern daher dadurch gekennzeichnet, dass es - bezogen auf das Gesamtgewicht des anwendungsbereiten Mittels - weniger als 0,5 Gew.-%, bevorzugt weniger als 0,3 Gew.-%, weiter bevorzugt weniger als 0,1 Gew.-% anionische Tenside und besonders bevorzugt keine anionischen Tenside enthält.

Als weiteren zusätzlichen Formulierungsbestandteil können die erfindungsgemäßen Mittel zusätzlich mindestens einen ethoxylierten Fettalkohol der Formel (III) mit einem Ethoxylierungsgrad von mindestens 25 enthalten.

Unter Ethoxylierung (auch Oxyethylierung) wird die Reaktion der Fettalkohole mit Ethylenoxid (EO) verstanden. Durch Insertion mindestens 25 Gruppierungen des Typs -CH2-CH2-O-pro Fettalkohol Molekül entstehen lineare Polyether, die an einem Kettenende eine Hydroxygruppe und am anderen Kettenende die C₈-C₂₈-Alkylgruppe des Fettalkohols tragen.

In einer weiteren besonders bevorzugten Ausführungsform ist ein erfindungsgemäßes Mittel dadurch gekennzeichnet, dass es zusätzlich einen oder mehrere ethoxylierte Fettalkohole der Formel (III) enthält, 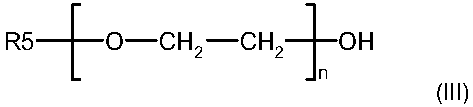 worin R5 für eine unverzweigte oder verzweigte, gesättige oder ungesättigte C₁₂-C₂₈-Alkylgruppe, bevorzugt für eine gesättigte, unverzweigte C₁₆- oder C₁₈- Alkylgruppe, steht und n für eine ganze Zahl von 25 bis 120, bevorzugt für eine ganze Zahl von 25 bis 80, weiter bevorzugt für eine ganze Zahl von 25 bis 50 und besonders bevorzugt für eine ganze Zahl von 25 bis 35 steht.

Bei einem ganz besonders bevorzugten ethoxylierten Fettalkohol der Formel (III) handelt es sich um Ceteareth-30.

Der oder die ethoxylierten Fettalkohole der Formel (III) können in den Mitteln in einer Gesamtmenge von 0,1 bis 2,0 Gew.-%, bevorzugt von 0,2 bis 1,6 Gew.-%, weiter bevorzugt von 0,3 bis 1,2 Gew.-% und besonders bevorzugt von 0,3 bis 0,6 Gew.-% - bezogen auf das Gesamtgewicht des anwendungsbereiten Mittels - enthalten sein.

Darüber hinaus hat es sich als besonders vorteilhaft herausgestellt, wenn die erfindungsgemäßen Mittel zusätzlich mindestens eine Kohlenwasserstoffverbindung enthalten. Insbesondere Paraffinum Liquidum hat sich in diesem Zusammenhang als geeignet erwiesen.

In einer weiteren bevorzugten Ausführungsform ist ein erfindungsgemäßes Mittel daher dadurch gekennzeichnet, dass es - bezogen auf das Gesamtgewicht des anwendungsbereiten Mittels - 0,5 bis 4,5 Gew.-%, bevorzugt 1,0 bis 4,0 Gew.-%, weiter bevorzugt 1,25 bis 3,0 Gew.-% und besonders bevorzugt 1,50 bis 2,0 Gew.-% Paraffinum Liquidum enthält. Wie bereits zuvor beschrieben handelt es sich bei den erfindungsgemäßen Mitteln um Mittel zum Blondieren bzw. Bleichen von keratinischen Fasern, diese Mittel enthalten in einer bevorzugten Ausführungsform keine Farbstoffe.

Zur Nuancierung der mit den Mitteln erhaltenden Blondnuance kann es jedoch unter Umständen notwendig werden, den Mitteln kleine Mengen an Oxidationsfarbstoffvorprodukten und/oder direktziehenden Farbstoffen zuzusetzen.

Unter die Oxidationsfarbstoffvorprodukte fallen Oxidationsfarbstoffvorprodukte vom Entwickler-Typ und vom Kuppler-Typ. Besonders geeignete Oxidationsfarbstoffvorprodukte vom Entwickler-Typ sind dabei ausgewählt aus mindestens einer Verbindung aus der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1 H-imidazol-1-yl)-propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind ausgewählt aus der Gruppe 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

Direktziehende Farbstoffe können in anionische, kationische und nichtionische direktziehende Farbstoffe unterteilt werden. Die direktziehenden Farbstoffe sind vorzugsweise ausgewählt aus den Nitrophenylendiaminen, den Nitroaminophenolen, den Azofarbstoffen, den Anthrachinonen, den Triarylmethanfarbstoffen oder den Indophenolen und deren physiologisch verträglichen Salzen.

Wenn die Oxidationsfarbsotffvorprodukte und/oder direktziehende Farbstoffe in den Mitteln enthalten sind, so beträgt ihre Gesamtmenge bevorzugt maximal 0,2 Gew.-%, weiter bevorzugt maximal 0,1 Gew.-% und besonders bevorzugt maximal 0,05 Gew.-%, bezogen auf das Gesamtgewicht des anwendungsbereiten Mittels.

Als Blondier- und Bleichmittel sind die erfindungsgemäßen Mittel weiterhin dadurch gekennzeichnet, dass sie Wasserstoffperoxid und/oder eines seiner festen Anlagerungsprodukte an organische oder anorganische Verbindungen enthalten.

In einer bevorzugten Ausführungsform wird Wasserstoffperoxid selbst als wässrige Lösung verwendet. Die Konzentration einer Wasserstoffperoxid-Lösung im erfindungsgemäßen Mittel wird einerseits von den gesetzlichen Vorgaben und andererseits von dem gewünschten Effekt bestimmt; vorzugsweise werden 6 bis 12 Gew.-%ige Lösungen in Wasser verwendet. Erfindungsgemäß bevorzugte anwendungsbereite Mittel des ersten Erfindungsgegenstands sind dadurch gekennzeichnet, dass sie, bezogen auf das Gesamtgewicht des anwendungsbereiten Mittels, 0,5 bis 20 Gew.-%, vorzugsweise 1 bis 12,5 Gew.-%, besonders bevorzugt 2,5 bis 10 Gew.-% und insbesondere 3 bis 6 Gew.-% Wasserstoffperoxid, jeweils bezogen auf das Gesamtgewicht des Mittels, enthalten.

Zur weiteren Steigerung der Aufhellung kann der erfindungsgemäßen Zusammensetzung zusätzlich mindestens eine SiO₂-Verbindung, wie Kieselsäure oder Silikate insbesondere Wassergläser, zugesetzt sein. Es kann erfindungsgemäß bevorzugt sein, die SiO₂-Verbindungen in Mengen von 8,0 Gew.-% bis 20,0 Gew.-%, besonders bevorzugt in Mengen von 10,0 Gew.-% bis 16 Gew.-% und ganz besonders bevorzugt in Mengen von 11,0 Gew.-% bis 14,0 Gew.-%, jeweils bezogen auf das anwendungsbereite Mittel, einzusetzen. Die Mengenangaben geben dabei jeweils den Gehalt der SiO₂-Verbindungen (ohne deren Wasseranteil) in den Mitteln wieder.

Die anwendungsbereiten Färbemittel können weiterhin zusätzliche Wirk-, Hilfs- und Zusatzstoffe enthalten, um die Färbeleistung zu verbessern und weitere gewünschte Eigenschaften der Mittel einzustellen.

Erfindungsgemäß geeignete Mittel können auch kationische Tenside vom Typ der quartären Ammoniumverbindungen, der Esterquats und der Amidoamine enthalten. Bevorzugte quaternäre Ammoniumverbindungen sind Ammoniumhalogenide sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Eine erfindungsgemäß besonders geeignete Verbindung aus der Amidoamine stellt das unter der Bezeichnung Tegoamid^{®} S 18 im Handel erhältliche Stearamidopropyl-dimethylamin dar. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Die kationischen Tenside sind in den erfindungsgemäß verwendeten Mitteln bevorzugt in Anteilen von 0,05 bis 10 Gew.-%, bezogen auf das gesamte Mittel, enthalten.

Die anwendungsbereiten Mittel können weitere Hilfs- und Zusatzstoffe enthalten. So hat es sich als vorteilhaft erwiesen, wenn die Mittel mindestens ein Verdickungsmittel enthalten. Bezüglich dieser Verdickungsmittel bestehen keine prinzipiellen Einschränkungen. Es können sowohl organische als auch rein anorganische Verdickungsmittel zum Einsatz kommen. Geeignete Verdickungsmittel sind
- kationische, synthetische Polymere;
- anionische, synthetische Polymere, wie Polyacrylate, Acrylates Copolymer, Copolymere von Acrylsäure und Methacrylsäure
- natürlich vorkommende Verdickungsmittel, wie nichtionische Guargums, Skleroglucangums oder Gummi arabicum, Ghatti-Gummi, Karaya-Gummi, Tragant-Gummi, Carrageen-Gummi, Johannisbrotkernmehl, Pektine, Xanthane, Alginate, Stärke-Fraktionen und Derivate wie Amylose, Amylopektin und Dextrine, sowie Cellulosederivate, wie beispielsweise Carboxymethylcellulose Methylcellulose und Hydroxyalkylcellulosen;
- nichtionische, vollsynthetische Polymere, wie Polyvinylalkohol oder Polyvinylpyrrolidinon; sowie
- anorganische Verdickungsmittel, insbesondere Schichtsilikate wie beispielsweise Bentonit, besonders Smektite, wie Montmorillonit oder Hectorit.

Ein besonders bevorzugtes kationisches Polymer ist Polyquaternium-4.

Bleich- und Blondierprozesse auf Keratinfasern laufen im alkalischen Bereich ab. Um die Keratinfasern und auch die Haut so weit wie möglich zu schonen, ist die Einstellung eines zu hohen pH-Wertes jedoch nicht wünschenswert. Der pH-Wert der erfindungsgemäßen Mittel kann daher zwischen 7,5 und 11, bevorzugt zwischen 9 und 10,5 liegen. Bei den pH-Werten im Sinne der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel werden bevorzugt aus Ammoniak, Alkanolaminen, basischen Aminosäuren sowie anorganischen Alkalisierungsmitteln ausgewählt. Bevorzugte anorganische Alkalisierungsmittel sind Magnesiumcarbonat, Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methyl-propanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin. Zur Einstellung des pH-Werts verwendbare Acificierungsmittel sind organische Säuren, wie Citronensäure, Essigsäure, Ascorbinsäure, Benzoesäure, Milchsäure, Äpfelsäure und Maleinsäure, sowie mineralische Säuren, wie Salzsäure, Schwefelsäure oder Phosphorsäure.

Weiterhin hat es sich als vorteilhaft erweisen, wenn die Färbemittel, insbesondere wenn sie zusätzlich Wasserstoffperoxid enthalten, mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Erfindungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol, insbesondere Polyquaternium-2, Polyquaternium-4, Polyquaternium-6, Polyquaternium-7, Polyquaternium-10, Polyquaternium-11, Polyquaternium-16, Polyquaternium-24, Polyquaternium-28, Polyquaternium-37, Polyquaternium-44, Polyquaternium-46, Polyquaternium-55, Polyquaternium-67, Polyquaternium-68, Polyquaternium-69 und Polyquaternium-87; zwitterionische und amphotere Polymere, wie insbesondere Polyquaternium-22 und Polyquaternium-39; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht der Anwendungsmischung, eingesetzt.

Zur Anwendung der erfindungsgemäßen Mittel eignet sich insbesondere ein Verfahren zum Blondieren von keratinischen Fasern, insbesondere menschlichen Haaren, welches dadurch gekennzeichnet ist, dass das anwendungsbereite Mittel erst kurz vor der Anwendung durch Vermischen von verschieden konfektionierten Zubereitungen hergestellt wird.Diese Anwendungsform gewährleistet, dass die für die Blondierwirkung verantwortlichen aktiven Spezies erst zum Zeitpunkt der Anwendung erzeugt werden.

In einer besonders bevorzugten Form wird das anwendungsbereite Mittel durch Vermischen von zwei separat verpackten Zubereitungen hergestellt, wobei es sich bei der ersten Zubereitung (Zubereitung A) um eine flüssige, gel- oder cremeförmige Komponente enthaltend Wasserstoffperoxid handelt. Aus Stabilitätsgründen ist diese Zubereitung schwach sauer bis sauer eingestellt. Bei der zweiten Zubereitung (Zubereitung B) handelt es sich bevorzugt um eine feste, pastöse oder pulverförmige Zubereitung, welche die Persulfate (Ammoniumpersulfat und gegebenenfalls weitere Persulfate) enthält. Diese Zubereitung enthält oft weiterhin Alkalisierungsmittel, bevorzugt in fester Form, wie beispielsweise Natriumsilikate (SiO2:Na2O im Verhältnis 2:1). Durch Vermengen der flüssig/gel/cremeförmigen Zubereitung A mit der festen/pastösen/pulvrigen Zubereitung B wird das anwendungsberiete Mittel hergestellt, welches einen alkalischen pH-Wert besitzt. Das Mischungsverhältnis der Zubereitungen A und B kann im Bereich von 1:1 zu 3:1 Gewichtsteilen liegen. Ganz besonders bevorzugt liegt das Mischungsverhältnis bei 2 Gewichtsteilen der Zubereitung A zu einem Gewichtsteil der Zubereitung B.

Das anwendungsbereite Mittel wird auf die keratinhaltigen Fasern aufgebracht, 5 bis 60 Minuten auf der Faser belassen und anschließend mit Wasser wieder ausgespült oder mit einem Shampoo ausgewaschen wird. Bevorzugt beträgt die Einwirkzeit der anwendungsbereiten Färbemittel 5 bis 45 min, insbesondere 10 bis 40 min, besonders bevorzugt 15 bis 35 min. Während der Einwirkzeit des Mittels auf der Faser kann es vorteilhaft sein, den Aufhellvorgang durch Wärmezufuhr zu unterstützen. Die Wärmezufuhr kann durch eine externe Wärmequelle, wie z.B. warme Luft eines Warmluftgebläses, als auch, insbesondere bei einer Haaraufhellung am lebenden Probanden, durch die Körpertemperatur des Probanden erfolgen. Bei letzterer Möglichkeit wird üblicherweise die aufzuhellende Partie mit einer Haube abgedeckt. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Nach Ende der Einwirkzeit wird die verbleibende Färbezubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Wasser erfolgen kann, wenn das Färbemittel einen stark tensidhaltigen Träger besitzt.

Ein weiterer Gegenstand der vorliegenden Erfindung ist ein anwendungsbereites Mittel zum Blondieren und/oder Bleichen von keratinischen Fasern, insbesondere menschlichen Haaren, dadurch gekennzeichnet, dass es unmittelbar vor der Anwendung durch Vermischen von zwei Zubereitungen (A) und (B) hergestellt wird, wobei
- es sich bei der Zubereitung (A) um ein Mittel handelt, das
   (a) Arachylalkohol (Eicosan-1-ol) und Behenylalkohol (Docosan-1-ol) in einer Gesamtmenge von 0,45 bis 5,1 Gew.-%, bevorzugt von 0,6 bis 3,9 Gew.-%, weiter bevorzugt von 0,75 bis 2,7 Gew.-% und besonders bevorzugt von 0,9 bis 1,35 Gew.-% - bezogen auf das Gesamtgewicht der Zubereitung (A) - enthält und
   (c) mindestens eine Verbindung ausgewählt aus Glycerylmonostearat, Glyceryldistearat, Glycerylmonopalmitat und Glyceryldipalmitat in einer Gesamtmenge von 0,15 bis 2,7 Gew.-%, bevorzugt 0,3 bis 2,0 Gew.-%, weiter bevorzugt von 0,4 bis 1,2 Gew.-% und besonders bevorzugt 0,45 bis 0,9 Gew.-% - bezogen auf das Gesamtgewicht der Zubereitung (A) - enthält,
- es sich bei der Zubereitung (B) um ein Mittel handelt, das Kaliumpersulfat und Ammoniumpersulfat in einer Gesamtmenge von 30 bis 54 Gew.-%, bevorzugt von 33 bis 51 Gew.-%, weiter bevrorzugt von 36 bis 48 Gew.-% und besonders bevorzugt von 39 bis 45 Gew.-% - bezogen auf das Gesamtgewicht der Zubereitung (B) - enthält, und
- das anwendungsbereite Mittel durch Vermischen von zwei Gewichtsteilen der Zubereitung (A) mit einem Gewichtsteil der Zubereitung (B) hergestellt wird.

Die zuvor beschriebenen Mittel eignen sich hervorragend zur komfortablen Blondierung von Haaren, da sie eine hohe Blondierleistung besitzen, der Anwender den Geruch des in den Mitteln enthaltenen Ammoniaks jedoch kaum wahrnimmt.

Ein weiterer Gegenstand der vorliegenden Erfindung ist daher die Verwendung der Kombination aus
(a) mindestens einen Fettalkohol ausgewählt aus der Gruppe Behenylalkohol (Docosan-1-ol), Erucylalkohol ((13Z)-Docos-13-en-1-ol) und Brassidylalkohol ((13E)-Docosen-1-ol) und
(c) mindestens einen Glycerylfettsäureester der allgemeinen Formel (I), 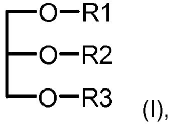 worin
   R1, R2 und R3 unabhängig voneinander für ein Wasserstoffatom oder eine Gruppierung der Formel (II) stehen, 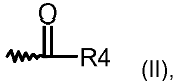 worin
   R4 für eine unverweigte oder verzweigte, gesättige oder ungesättigte C₁₁-C₂₇-Alkylgruppe steht,
   mit der Maßgabe, dass mindestens einer und maximal zwei der Reste ausgewählt aus R1, R2 und R3 für eine Gruppierung der Formel (II) stehen, zur Reduktion des Ammoniakgeruchs von Blondiermitteln, welche in einem kosmetischen Träger
(b) Ammoniumpersulfat sowie gegebenenfalls ein weiteres Persulfatsalz enthalten.

### 1. Formulierunasbeispiele

Es wurden die folgenden Rezepturen hergestellt. Die Mengenangaben verstehen sich, sofern nichts anderes vermerkt ist, jeweils in Gewichtsprozent.

**Zubereitung A**

| Formulierungsbestandteile | (Gew.-%) |
|---|---|
| Cetarylalkohol | 3,70 |
| Behenylalkohol (Docosan-1-ol) | 1,10 |
| Paraffinium Liquidum | 0,65 |
| Ceteareth-30 (C₁₆-C₁₈ Fettalkohole, ethoxyliert mit 30 EO) | 0,50 |
| Cutina GMS-V (Gemisch aus Glycerylmonostearat, Glyceryldistearat, Glycerylmonopalmitat und Glyceryldipalmitat) | 0,50 |
| Hydroxyethan-1,1-diposphonsäure | 0,15 |
| Propylenglycol | 0,50 |
| Cocamidopropylbetain | 0,12 |
| Dinatriumpyrophosphat | 0,18 |
| Phenoxyethanol | 0,20 |
| Natriumbenzoat | 0,10 |
| Wasserstoffperoxid (50 %ige wässrige Lösung) | 12,00 |
| Wasser | ad 100 |

**Zubereitung B**

| Formulierungsbestandteile | (Gew.-%) |
|---|---|
| Natriumsilikat (SiO₂:Na₂O = 2:1) | 36,00 |
| Magnesiumcarbonat | 12,85 |
| Natriumhexametaphosphat | 0,20 |
| Rohagit S hv (Methylmethacrylat, Methacrylsäure Copolymer) | 1,00 |
| EDTA Dinatriumsalz (Ethylenediaminetetraessigsäure, Dinatriumsalz) | 0,60 |
| Polyquaternium-4 | 0,30 |
| Kieselsäure, hydrophil | 0,40 |
| Glycin | 0,60 |
| Kaliumpersulfat | 32,00 |
| Ammoniumpersulfat | 10,00 |
| Ariabel Blue 300302 (CI 77007 (ULTRAMARINES)) | 0,15 |
| Dimeticone, Dimethiconol | 1,50 |
| Paraffinium Liquidum | 3,80 |

Zur Herstellung des anwendungsbereiten Mittels wurden 2 Gewichtsteile der Zubereitung A mit einem Gewichtsteil der Zubereitung B vermischt.

## Patentansprüche

1. Anwendungsbereites Mittel zum Blondieren und/oder Bleichen von keratinischen Fasern, insbesondere menschlichen Haaren, das durch Vermischen von zwei separat verpackten Zubereitungen hergestellt wird, wobei es sich bei der ersten Zubereitung (Zubereitung A) um eine flüssige, gel- oder cremeförmige Komponente enthaltend Wasserstoffperoxid handelt, die aus Stabilitätsgründen schwach sauer bis sauer eingestellt ist, und es sich bei der zweiten Zubereitung (Zubereitung B) um eine feste, pastöse oder pulverförmige Zubereitung handelt, welche die Persulfate und weiterhin Alkalisierungsmittel enthält, wobei
das anwendungsbereite Mittel einen alkalischen pH-Wert besitzt und in einem kosmetischen Träger enthält
(a) mindestens einen Fettalkohol ausgewählt aus der Gruppe Behenylalkohol (Docosan-1-ol), Erucylalkohol ((13Z)-Docos-13-en-1-ol) und Brassidylalkohol ((13*E*)-Docosen-1-ol),
(b) Ammoniumpersulfat sowie gegebenenfalls ein weiteres Persulfatsalz,
(c) mindestens einen Glycerylfettsäureester der allgemeinen Formel (I), 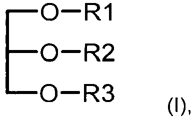 worin
R1, R2 und R3 unabhängig voneinander für ein Wasserstoffatom oder eine Gruppierung der Formel (II) stehen, 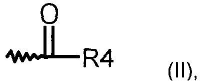 worin
R4 für eine unverzweigte oder verzweigte, gesättigte oder ungesättigte C₁₁-C₂₇-Alkylgruppe steht, mit der Maßgabe, dass mindestens einer und maximal zwei der Reste ausgewählt aus R1, R2 und R3 für eine Gruppierung der Formel (II) stehen.

2. Mittel nach Anspruch 1, **dadurch gekennzeichnet, dass** es als Fettalkohol(e) (a) Behenylalkohol (Docosan-1-ol) in einer Gesamtmenge von 0,3 bis 3,4 Gew.-%, bevorzugt von 0,4 bis 2,6 Gew.-%, weiter bevorzugt von 0,5 bis 1,8 Gew.-% und besonders bevorzugt von 0,6 bis 0,9 Gew.-% - bezogen auf das Gesamtgewicht des anwendungsbereiten Mittels - enthält.

3. Mittel nach einem der Ansprüche 1 oder 2, **dadurch gekennzeichnet, dass** die Gesamtmenge aller im Mittel enthaltenen Fettalkohole - bezogen auf das Gesamtgewicht des anwendungsbereiten Mittels - eine Menge von 5,5 Gew.-%, bevorzugt eine Menge von 4,8 Gew.-%, weiter bevorzugt eine Menge von 4,1 Gew.-% und besonders bevorzugt eine Menge von 3,5 Gew.-% nicht überschreitet.

4. Mittel nach einem der Ansprüche 1 bis 3, **dadurch gekennzeichnet, dass** es als Persulfatsalze (b) Kaliumpersulfat und Ammoniumpersulfat in einer Gesamtmenge von 10 bis 18 Gew.-%, bevorzugt von 11 bis 17 Gew.-%, weiter bevorzugt von 12 bis 16 Gew.-% und besonders bevorzugt von 13 bis 15 Gew.-% - bezogen auf das Gesamtgewicht des anwendungsbereiten Mittels - enthält.

5. Mittel nach einem der Ansprüche 1 bis 4, **dadurch gekennzeichnet, dass** es als Persulfatsalze (b) Kaliumpersulfat und Ammoniumpersulfat enthält, wobei das Gewichtsverhältnis von Kaliumpersulfat zu Ammoniumpersulfat im Bereich von 1:1 bis 6:1, bevorzugt von 2:1 bis 5:1 und besonders bevorzugt von 3:1 bis 4:1 - bezogen auf das Gesamtgewicht des anwendungsbereiten Mittels - liegt.

6. Mittel nach einem der Ansprüche 1 bis 5, **dadurch gekennzeichnet, dass** es als Glycerylfettsäureester (c) der allgemeinen Formel (I) mindestens eine Verbindung ausgewählt aus Glycerylmonostearat, Glyceryldistearat, Glycerylmonopalmitat und Glyceryldipalmitat in einer Gesamtmenge von 0,1 bis 1,8 Gew.-%, bevorzugt von 0,2 bis 1,3 Gew.-%, weiter bevorzugt von 0,25 bis 0,8 Gew.-% und besonders bevorzugt von 0,3 bis 0,6 Gew.-% - bezogen auf das Gesamtgewicht des anwendungsbereiten Mittels - enthält.

7. Mittel nach einem der Ansprüche 1 bis 6, **dadurch gekennzeichnet, dass** es zusätzlich ein oder mehrere amphotere und/oder zwitterionische Tenside in einer Gesamtmenge von 0,01 bis 0,5 Gew.-%, bevorzugt von 0,03 bis 0,4 Gew.-%, weiter bevorzugt von 0,05 bis 0,3 Gew.-% und besonders bevorzugt von 0,07 bis 0,2 Gew.-% - bezogen auf das Gesamtgewicht des anwendungsbereiten Mittels - enthält.

8. Mittel nach einem der Ansprüche 1 bis 7, **dadurch gekennzeichnet, dass** es - bezogen auf das Gesamtgewicht des anwendungsbereiten Mittels - weniger als 0,5 Gew.-%, bevorzugt weniger als 0,3 Gew.-%, weiter bevorzugt weniger als 0,1 Gew.-% anionische Tenside und besonders bevorzugt keine anionischen Tenside enthält.

9. Mittel nach einem der Ansprüche 1 bis 8, **dadurch gekennzeichnet, dass** es zusätzlich einen oder mehrere ethoxylierte Fettalkohole der Formel (III) enthält, 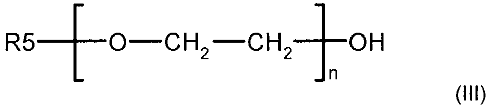 R5 für eine unverzweigte oder verzweigte, gesättigte oder ungesättigte C₁₂-C₂₈-Alkylgruppe, bevorzugt für eine gesättigte, unverzweigte C₁₆- oder C₁₈- Alkylgruppe, steht und
n für eine ganze Zahl von 25 bis 120, bevorzugt für eine ganze Zahl von 25 bis 80, weiter bevorzugt für eine ganze Zahl von 25 bis 50 und besonders bevorzugt für eine ganze Zahl von 25 bis 35 steht.

10. Anwendungsbereites Mittel laut Anspruch 1 zum Blondieren und/oder Bleichen von keratinischen Fasern,
insbesondere menschlichen Haaren, **dadurch gekennzeichnet, dass** es unmittelbar vor der Anwendung durch Vermischen von zwei Zubereitungen (A) und (B) hergestellt wird, wobei
- es sich bei der Zubereitung (A) um ein Mittel handelt, das
(a) Arachylalkohol (Eicosan-1-ol) und/oder Behenylalkohol (Docosan-1-ol) in einer Gesamtmenge von 0,45 bis 5,1 Gew.-%, bevorzugt von 0,6 bis 3,9 Gew.-%, weiter bevorzugt von 0,75 bis 2,7 Gew.-% und besonders bevorzugt von 0,9 bis 1,35 Gew.-% - bezogen auf das Gesamtgewicht der Zubereitung (A) - enthält, wobei das Mittel Behenylalkohol (Docosan-1-ol) enthält, und
(c) mindestens eine Verbindung ausgewählt aus Glycerylmonostearat, Glyceryldistearat, Glycerylmonopalmitat und Glyceryldipalmitat in einer Gesamtmenge von 0,15 bis 2,7 Gew.-%, bevorzugt 0,3 bis 2,0 Gew.-%, weiter bevorzugt von 0,4 bis 1,2 Gew.-% und besonders bevorzugt 0,45 bis 0,9 Gew.-% - bezogen auf das Gesamtgewicht der Zubereitung (A) - enthält,
- es sich bei der Zubereitung (B) um ein Mittel handelt, das Kaliumpersulfat und Ammoniumpersulfat in einer Gesamtmenge von 30 bis 54 Gew.-%, bevorzugt von 33 bis 51 Gew.-%, weiter bevorzugt von 36 bis 48 Gew.-% und besonders bevorzugt von 39 bis 45 Gew.-% - bezogen auf das Gesamtgewicht der Zubereitung (B) - enthält, und
- das anwendungsbereite Mittel durch Vermischen von zwei Gewichtsteilen der Zubereitung (A) mit einem Gewichtsteil der Zubereitung (B) hergestellt wird.

## Claims

1. A ready-to-use agent for dyeing blond and/or bleaching keratin fibers, in particular human hair, which agent is produced by mixing two separately packaged preparations, wherein the first preparation (preparation A) is a liquid, gelatinous or creamy component containing hydrogen peroxide, which component is weakly acidic to acidic for stability reasons, and the second preparation (preparation B) is a solid, pasty or powdered preparation which contains the persulfates and also alkalizing agents, wherein the ready-to-use agent has an alkaline pH and contains, in a cosmetic carrier,
(a) at least one fatty alcohol selected from the group consisting of behenyl alcohol (docosan-1-ol), erucyl alcohol ((13Z)-docos-13-en-1-ol) and brassidyl alcohol ((13E)-docosen-1-ol),
(b) ammonium persulfate as well as optionally a further persulfate salt,
(c) at least one glyceryl fatty acid ester of general formula (I), 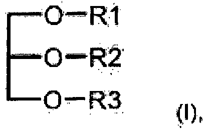 where
R1, R2 and R3, independently of one another, represent a hydrogen atom or a grouping of formula (II), 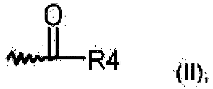 where
R4 represents an unbranched or branched, saturated or unsaturated C₁₁-C₂₇ alkyl group, with the proviso that at least one and no more than two of the functional groups selected from R1, R2 and R3 represent a grouping of formula (II).

2. The agent according to claim 1, **characterized in that** it contains (a) behenyl alcohol (docosan-1-ol) as the fatty alcohol(s) in a total amount of from 0.3 to 3.4 wt.%, preferably from 0.4 to 2.6 wt.%, more preferably from 0.5 to 1.8 wt.% and particularly preferably from 0.6 to 0.9 wt.%, based on the total weight of the ready-to-use agent.

3. The agent according to one of claims 1 or 2, **characterized in that** the total amount of all the fatty alcohols contained in the agent, based on the total weight of the ready-to-use agent, does not exceed an amount of 5.5 wt.%, preferably an amount of 4.8 wt.%, more preferably an amount of 4.1 wt.% and particularly preferably an amount of 3.5 wt.%.

4. The agent according to one of claims 1 to 3, **characterized in that** it contains potassium persulfate and ammonium persulfate as the persulfate salts (b) in a total amount of from 10 to 18 wt.%, preferably from 11 to 17 wt.%, more preferably from 12 to 16 wt.% and particularly preferably from 13 to 15 wt.%, based on the total weight of the ready-to-use agent.

5. The agent according to one of claims 1 to 4, **characterized in that** it contains potassium persulfate and ammonium persulfate as the persulfate salts (b), the weight ratio of potassium persulfate to ammonium persulfate being in the range of from 1:1 to 6:1, preferably from 2:1 to 5:1 and particularly preferably from 3:1 to 4:1, based on the total weight of the ready-to-use agent.

6. The agent according to one of claims 1 to 5, **characterized in that** it contains, as the glyceryl fatty acid esters (c) of general formula (I), at least one compound selected from glyceryl monostearate, glyceryl distearate, glyceryl monopalmitate and glyceryl dipalmitate, in a total amount of from 0.1 to 1.8 wt.%, preferably from 0.2 to 1.3 wt.%, more preferably from 0.25 to 0.8 wt.% and particularly preferably from 0.3 to 0.6 wt.%, based on the total weight of the ready-to-use agent.

7. The agent according to one of claims 1 to 6, **characterized in that** it additionally contains one or more amphoteric and/or zwitterionic surfactants in a total amount of from 0.01 to 0.5 wt.%, preferably from 0.03 to 0.4 wt.%, more preferably from 0.05 to 0.3 wt.% and particularly preferably from 0.07 to 0.2 wt.%, based on the total weight of the ready-to-use agent.

8. The agent according to one of claims 1 to 7, **characterized in that** it contains, based on the total weight of the ready-to-use agent, less than 0.5 wt.%, preferably less than 0.3 wt.%, more preferably less than 0.1 wt.% anionic surfactants and particularly preferably no anionic surfactants.

9. The agent according to one of claims 1 to 8, **characterized in that** it additionally contains one or more ethoxylated fatty alcohols of formula (III) 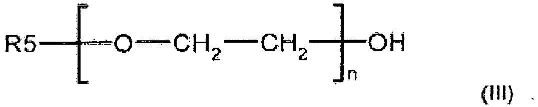 where
R5 represents an unbranched or branched, saturated or unsaturated C₁₂-C₂₈ alkyl group, preferably a saturated, unbranched C₁₆ or C₁₈ alkyl group, and
n represents an integer of from 25 to 120, preferably an integer of from 25 to 80, more preferably an integer of from 25 to 50 and particularly preferably an integer of from 25 to 35.

10. The ready-to-use agent according to claim 1 for dyeing blond and/or bleaching keratin fibers, in particular human hair, **characterized in that** said agent is produced immediately prior to application by mixing two preparations (A) and (B),
- preparation (A) being an agent which
(a) contains arachyl alcohol (eicosan-1-ol) and/or behenyl alcohol (docosan-1-ol) in a total amount of from 0.45 to 5.1 wt.%, preferably from 0.6 to 3.9 wt.%, more preferably from 0.75 to 2.7 wt.% and particularly preferably from 0.9 to 1.35 wt.%, based on the total weight of preparation (A), the agent containing behenyl alcohol (docosan-1-ol), and
(c) contains at least one compound selected from glyceryl monostearate, glyceryl distearate, glyceryl monopalmitate and glyceryl dipalmitate in a total amount of from 0.15 to 2.7 wt.%, preferably from 0.3 to 2.0 wt.%, more preferably from 0.4 to 1.2 wt.% and particularly preferably from 0.45 to 0.9 wt.%, based on the total weight of preparation (A),
- preparation (B) being an agent that contains potassium persulfate and ammonium persulfate in a total amount of from 30 to 54 wt.%, preferably from 33 to 51 wt.%, more preferably from 36 to 48 wt.% and particularly preferably from 39 to 45 wt.%, based on the total weight of preparation (B), and
- the ready-to-apply agent being produced by mixing two parts by weight of preparation (A) with one part by weight of preparation (B).

## Revendications

1. Agent prêt à l'emploi pour la coloration en blond et/ou l'éclaircissement de fibres kératiniques, notamment de cheveux humains, produit par le mélange de deux préparations conditionnées séparément, dans lequel la première préparation (préparation A) est un composant liquide, sous forme de gel ou de crème contenant du peroxyde d'hydrogène, qui est faiblement acide à acide pour des raisons de stabilité, et la seconde préparation (préparation B) est une préparation solide, sous forme de pâte ou sous forme de poudre, laquelle contient les persulfates et en outre un agent d'alcalinisation, dans lequel l'agent prêt à l'emploi possède une valeur pH alcaline et contient dans un support cosmétique
(a) au moins un alcool gras sélectionné parmi le groupe de l'alcool béhénylique (docosan-1-ol), l'alcool érucylique ((13*Z*)-Docos-13-en-1-ol) et l'alcool brassidylique ((13*E*)-Docosen-1-ol),
(b) du persulfate d'ammonium ainsi que le cas échéant un autre sel de persulfate,
(c) au moins un ester d'acide gras glycérique de la formule générale (I), 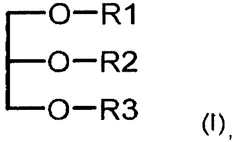 dans laquelle
R1, R2 et R3 représentent indépendamment l'un de l'autre un atome d'hydrogène ou un groupement de la formule (II), 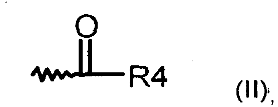 dans laquelle
R4 représente un groupe alkyle en C₁₁-C₂₇ non-ramifié ou ramifié, saturé ou insaturé, à la condition qu'au moins un et au maximum deux des résidus sélectionnés parmi R1, R2 et R3 représentent un groupement de la formule (II).

2. Agent selon la revendication 1, **caractérisé en ce qu'**il contient en tant qu'alcool(s) gras (a) de l'alcool béhénylique (docosan-1-ol) dans une quantité totale de 0,3 à 3,4 % en poids, préférablement de 0,4 à 2,6 % en poids, plus préférablement de 0,5 à 1,8 % en poids et très préférablement de 0,6 à 0,9 % en poids - rapporté au poids total de l'agent prêt à l'emploi.

3. Agent selon l'une quelconque des revendications 1 ou 2, **caractérisé en ce que** la quantité totale de tous les alcools gras contenus dans l'agent- rapporté au poids total de l'agent prêt à l'emploi - ne dépasse pas une quantité de 5,5 % en poids, préférablement une quantité de 4,8 % en poids, plus préférablement une quantité de 4,1 % en poids et très préférablement une quantité de 3,5 % en poids.

4. Agent selon l'une quelconque des revendications 1 à 3 **caractérisé en ce qu'**il contient-rapporté au poids total de l'agent prêt à l'emploi - en tant que sels de persulfates (b) du persulfate de potassium et du persulfate d'ammonium dans une quantité totale de 10 à 18 % en poids, préférablement de 11 à 17 % en poids, plus préférablement de 12 à 16 % en poids et très préférablement de 13 à 15 % en poids.

5. Agent selon l'une quelconque des revendications 1 à 4, **caractérisé en ce qu'**il contient du persulfate de potassium et du persulfate d'ammonium en tant que sels de persulfate (b), dans lequel le rapport de poids de persulfate de potassium sur persulfate d'ammonium se trouve dans une plage de 1:1 à 6:1, préférablement de 2:1 à 5:1 et très préférablement de 3:1 à 4:1 - rapporté au poids total de l'agent prêt à l'emploi.

6. Agent selon l'une quelconque des revendications 1 à 5, **caractérisé en ce qu'**il contient en tant qu'ester d'acide gras glycérique (c) de la formule générale (I) au moins un composé sélectionné parmi le monostéarate de glycéryle, le distéarate de glycéryle, le monopalmitate de glycéryle et le dipalmitate de glycéryle dans une quantité totale de 0,1 à 1,8 % en poids, préférablement de 0,2 à 1,3 % en poids, plus préférablement de 0,25 à 0,8 % en poids et très préférablement de 0,3 à 0,6 % en poids - rapporté au poids total de l'agent prêt à l'emploi.

7. Agent selon l'une quelconque des revendications 1 à 6, **caractérisé en ce qu'**il contient en outre un ou plusieurs tensioactifs amphotères et/ou zwitterioniques dans une quantité totale de 0,01 à 0,5 % en poids, préférablement de 0,03 à 0,4 % en poids, plus préférablement de 0,05 à 0,3 % en poids et très préférablement de 0,07 à 0,2 % en poids - rapporté au poids total de l'agent prêt à l'emploi.

8. Agent selon l'une quelconque des revendications 1 à 7, **caractérisé en ce qu'**il contient - rapporté au poids total de l'agent prêt à l'emploi - moins de 0,5 % en poids, préférablement moins de 0,3 % en poids, plus préférablement moins de 0,1 % en poids en tensioactifs anioniques et très préférablement ne contient aucun tensioactif anionique.

9. Agent selon l'une quelconque des revendications 1 à 8, **caractérisé en ce qu'**il contient en outre un ou plusieurs alcool(s) gras éthoxylés de la formule (III) 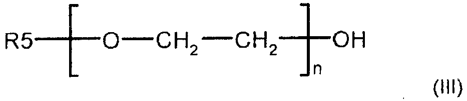 dans laquelle
R5 représente un groupe alkyle en C₁₂-C₂₈ non-ramifié ou ramifié, saturé ou insaturé, préférablement un groupe alkyle en C₁₆ ou en C₁₈ saturé, non-ramifié, et
n représente un nombre entier de 25 à 120, préférablement un nombre entier de 25 à 80, plus préférablement un nombre entier de 25 à 50 et très préférablement un nombre entier de 25 à 35.

10. Agent prêt à l'emploi pour la coloration en blond et/ou l'éclaircissement de fibres kératiniques, notamment de cheveux humains, selon la revendication 1, **caractérisé en ce qu'**il est produit immédiatement avant utilisation par le mélange de deux préparations (A) et (B), dans lequel
- la préparation (A) est un agent qui
(a) contient de l'alcool arachylique (eicosan-1-ol) et/ou de l'alcool béhénylique (docosan-1-ol) dans une quantité totale de 0,45 à 5,1 % en poids, préférablement de 0,6 à 3,9 % en poids, plus préférablement de 0,75 à 2,7 % en poids et très préférablement de 0,9 à 1,35 % en poids - rapporté au poids total de la préparation (A), dans lequel l'agent contient de l'alcool béhénylique (docosan-1-ol), et
(c) contient au moins un composé sélectionné parmi le monostéarate de glycéryle, le distéarate de glycéyle, le monopalmitate de glycéryle et le dipalmitate de glycéryle dans une quantité totale de 0,15 à 2,7 % en poids, préférablement de 0,3 à 2,0 % en poids, plus préférablement de 0,4 à 1,2 % en poids et très préférablement de 0,45 à 0,9 % en poids - rapporté au poids total de la préparation (A),
- la préparation (B) est un agent qui contient du persulfate de potassium et du persulfate d'ammonium dans une quantité totale de 30 à 54 % en poids, préférablement de 33 à 51 % en poids, plus préférablement de 36 à 48 % en poids et très préférablement de 39 à 45 % en poids
- rapporté au poids total de la préparation (B), et
- l'agent prêt à l'emploi est produit par mélange de deux parties en poids de la préparation (A) avec une partie en poids de la préparation (B).
